# EUROPÄISCHE PATENTSCHRIFT

(11) **EP 1 448 252 B1**
(45) Veröffentlichungstag und Bekanntmachung des Hinweises auf die Patenterteilung: **18.04.2007**
(21) Anmeldenummer: 02781512.5
(22) Anmeldetag: 15.11.2002
(51) Int. Cl.: A61M 5/142

(54) **MEDIZINISCHE PUMPVORRICHTUNG**
MEDICAL PUMP DEVICE
ENSEMBLE POMPE MEDICALE

(30) Priorität: 16.11.2001 CH 20012110; 22.05.2002 CH 20020857
(43) Veröffentlichungstag der Anmeldung: 25.08.2004
(73) Patentinhaber: Medinnovation AG, 8003 Zürich (CH)
(72) Erfinder: FÜCHSLIN, Rudolf, Marcel, CH-5200 Brugg (CH); WEISS, Markus, CH-8494 Bauma (CH); DÜNKI, Rudolf, CH-8406 Winterthur (CH); MEIER, Peter, Fritz, CH-5000 Aarau (CH); NEFF, Thomas, CH-8708 Männedorf (CH); STOLL, Erich, Paul, CH-8143 Stallikon (CH); SUTER, Andreas, CH-5303 Würenlingen (CH)
(74) Vertreter: Blum, Rudolf Emil
(86) Internationale Anmeldenummer: PCT/IB2002/004771
(87) Internationale Veröffentlichungsnummer: WO 2003/041767

(56) Entgegenhaltungen:
- EP-A- 0 345 396
- EP-A- 1 020 203
- DE-A- 19 909 654
- GB-A- 2 060 131
- GB-A- 2 157 374
- US-A- 4 798 589

## Beschreibung

### Technisches Gebiet

Die Erfindung betrifft eine medizinische Pumpvorrichtung gemäss Oberbegriff des Anspruchs 1. Ferner betrifft die Erfindung ein Pumpsystem gemäss Anspruch 8.

### Stand der Technik

Zur kontinuierlichen Applikation von Flüssigkeiten oder Medikamenten werden Infusionspumpen und Spritzenpumpen verwendet. Während bei den Infusionspumpen Flüssigkeiten mittels einer auf einen Schlauch wirkenden Quetschwalze vorangetrieben werden, wird bei der Spritzenpumpe die Medikamentenlösung durch Verschieben des Spritzenkolbens ausgestossen. Bei letzterem System ist die Menge eines ausgestossenen Medikamentes eine Funktion der Position dieses Kolbens. Spritzenpumpensysteme werden vor allem eingesetzt, um potente Medikamente kontinuierlich als hochkonzentrierte Lösung bei niedrigen Flussraten zu applizieren.

Vor allem in der pädiatrischen Intensivmedizin wird mit sehr tiefen Flussraten gearbeitet, um die Patienten nicht unnötig mit Flüssigkeit zu belasten. Zu grosse Schwankungen der Flussrate können bei der Anwendung von hochkonzentrierten, potenten, kurzwirksamen Medikamenten lebensgefährlich sein.

Spritzenpumpensysteme sind bei tiefen Flussraten sehr empfindlich auf hydrostatische Druckänderungen (Anheben/Absenken der Pumpe) bedingt durch die Nachgiebigkeit von Pumpe, Spritze (insbesondere Kolbengummi), Luft in der Flüssigkeitslösung und Infusionsleitung [Literatur 1,2 gemäss Literaturverzeichnis am Ende der Beschreibung]. Elastische Deformationen und kurzzeitiges Verklemmen (Sticking-Effekt) führen dazu, dass keine genaue Relation zwischen Kolbenposition und ausgestossenem Volumen besteht. Eine vertikale Senkung der Spritzenpumpe kann aufgrund der Änderung der hydrostatischen Druckverhältnisse zu Siphoneffekten mit retrogradem Fluss und Nullmedikamentenapplikationszeit führen. Dies wird signifikant vor allem bei geringen Flussraten (< 1 ml/h). Umgekehrt kann das Anheben der Pumpe zu einem Entleeren (Infusionsbolus) der dehnbaren Elemente führen [3,4].

Bei Verschluss oder Knicken der Leitung verzögern die dehnbaren Elemente den Druckaufbau im Infusionssystem und damit den Verschlussalarm [10-12].

Die eingehende Analyse der einzelnen Komponenten hat ergeben, dass der Hauptanteil der Nachgiebigkeit vor allem von der Spritzenpumpe, der Infusionsspritze und der Luft herrührt [5-9, 12].

Dabei konnte gezeigt werden, dass die Dehnbarkeit dünner, steifer Infusionsleitungen weitaus geringer ist als diejenige des Reservoirs, d.h. der Pumpe und der Spritze.

Infusionspumpen andererseits, wie z.B. aus US-A-6 213 723 bekannt, eigenen sich in der Regel weniger für die Applikation von hochkonzentrierten, kurzwirksamen Medikamenten bei tiefen Flussraten. Das Einsetzen der proximalen Quetsche vor dem Lösen der distalen Quetsche führt zu einem Druckanstieg in der Infusionsleitung, welcher beim Lösen der distalen Quetsche zu einer kurzzeitigen hohen Flussrate führt. Die dazu verwendeten Infusionssysteme sind hoch-dehnbar und damit äusserst empfindlich auf hydrostatische Änderungen. Beide Infusionssysteme haben den Nachteil, dass sie keine Kontrolle über die effektiv exakt abgegebene Flüssigkeitsmenge bieten [13-16].

Aus US-A-4 273 121 ist ferner eine Kassettenpumpe bekannt, die zwischen einem Infusionsbehälter und dem Katheter bzw. Patienten angeordnet wird. Die Kassette enthält eine von einer Membran begrenzte Kammer, wobei die Druckbeaufschlagung der Membran im Zusammenspiel mit eingangsseitig und ausgangsseitig angeordneten, als Ventile wirkenden Schlauchklemmen das Pumpen von geringen Volumina Richtung Patient ermöglicht. Da indes bei dieser Pumpe bis zu 75 % der nachgiebigen Membranfläche frei liegt und vom zugehörigen Stössel nicht beaufschlagt wird, ergibt sich ebenfalls eine erhebliche Abhängigkeit dieser Kassettenpumpe vom Eingangsdruck der Flüssigkeit und damit der Höhenlage des Infusionsbehälters. Ein Zusammenwirken mit einer Spritzenpumpe als Speisung der Kassettenpumpe ist nicht erwähnt und erscheint aufgrund der Nachgiebigkeit und entsprechender Lageabhängigkeit beider Pumpen nicht vorteilhaft.

Aus US-A-4 798 589, GB-A-2 060 131 und EP-A-0 345 396 sind medizinische Pumpsysteme gemäss Oberbegriff des Anspruchs 1 bekannt.

### Darstellung der Erfindung

Der Erfindung liegt die Aufgabe zugrunde, eine Pumpvorrichtung und ein Pumpsystem zu schaffen, welches einen besonders einfachen Aufbau aufweist.

Dies wird bei einer Pumpvorrichtung der eingangs genannten Art mit den Merkmalen des Anspruchs 1 erzielt sowie mit einem Pumpsystem nach Anspruch 8.

Durch das Drehventil mit gleichmässig verteilten Anschlüssen und Pumpenkammer ergibt sich ein sehr einfacher Aufbau. Der geradlinige Kanal ermöglicht ein sicheres Spülen der Vorrichtung und vermeidet Vorsprünge an denen Luftblasen ansetzen können. Vorzugsweise ist nur ein einzelner Kanal vorgesehen.

Durch die bevorzugte starre Ausbildung der Pumpenkammer kann die Pumpvorrichtung unabhängig von Eingangsdruckschwankungen immer dasselbe Pumpenvolumen fördern; entsprechendes gilt für das Pumpsystem, bei dem die Nachgiebigkeit der Infusionsspritze und des Treibers keinen Einfluss mehr auf das geförderte Pumpvolumen hat.

Bei einer bevorzugten Ausführungsform wird die starre aber bewegliche Kammerwand von einem starren Kolben oder Stössel gebildet. Bei einer anderen bevorzugten Ausführungsform wird die starre Kammerwand durch eine an sich elastische oder eine quasi-elastische Membran gebildet. Diese kann vollständig von einem starren Betätigungsmittel beaufschlagt werden, vorzugsweise einer Flüssigkeit, so dass die Membran ebenfalls als starres Element wirkt, das nur soweit nachgibt, wie es das inkompressible Betätigungsmittel erlaubt, so dass volumenkonstant gepumpt werden kann. Unter einer quasi-elastischen Membran wird dabei eine Membran verstanden, deren Ausdehnung durch eine Armierung begrenzt wird.

Es ergibt sich somit ein Infusionssystem mit minimaler Nachgiebigkeit, welches erlaubt die abgegebene Flüssigkeitsmenge zu überwachen. Dazu wird ein vorzugsweise nicht-dehnbarer, mikro-volumetrisch kontrollierbarer Infusionstreiber zwischen dehnbarem Treiber mit Infusionsspritze oder Infusionsbehälter und im wesentlichen nicht-dehnbarer Infusionsleitung zwischengeschaltet. Prinzipiell werden kleine Volumina von exakt definierter Grösse V und minimaler Nachgiebigkeit zyklisch gefüllt und vollständig entleert. Zu jedem Zeitpunkt ist dann die total applizierte Medikamentenmenge bis auf ein Volumen V bekannt. Die Entleerungen der Volumina V können gezählt werden. Der Pumpprozess wird diskretisiert. Ungenauigkeiten können nur zwei Quellen haben. Erstens kann man sich gerade in einem Entleerungsprozess befinden, was eine maximale Ungenauigkeit von V ergibt. In den vorgeschlagenen vorteilhaften Realisierungen beträgt V 1-10 mm³, was die erwähnte Ungenauigkeit auf ein medizinisch unbedeutendes Mass reduziert. Zweitens hat man eine additive, d.h. mit der Zeit anwachsende Ungenauigkeit durch die Nachgiebigkeit der Volumina V selber. Diese wird jedoch in den nachfolgend vorgeschlagenen Systemen als klein vorausgesetzt.

Das vorgeschlagene Prinzip ermöglicht es insbesondere, Volumeneffekte, welche durch Veränderungen des hydrostatischen Druckes verursacht werden, weitestgehend zu reduzieren, da der Flüssigkeitsvorschub nicht mehr eigentlich durch den applizierten Druck auf ein grösseres Volumen, sondern durch die Anzahl Entleerungen der Volumina V bestimmt wird.

Grundsätzlich weist das vorgeschlagene Infusionssystem ein Flüssigkeitsreservoir, einen Infusionstreiber (Volumendiskretisator) und eine nachgeschaltete starre Infusionsleitung auf, wobei das Flüssigkeitsreservoir durch die vertikale Höhe oder durch eine Spannfeder unter leichtem Druck steht. Der Infusionstreiber besteht vorteilhafterweise aus einem wegwerfbaren Plastikteil, einem wiederverwendbaren Antriebsteil und einer Steuereinheit.

Die Vorrichtung zum volumenkontrollierten Pumpen eines flüssigen Mediums im Bereiche medizinischer Anwendungen erlaubt also die Kontrolle der Flussrate durch Diskretisieren des entleerbaren Flüssigkeitsvolumen unter Verwendung zyklisch füll- und entleerbarer Volumina mit genau definiertem Inhalt. Die Position der Vorrichtung ist zwischen Reservoir und Patient irgendwo in der Leitung. Bevorzugt ist eine Bypassfunktion zur Entlüftung des Systems. Bevorzugt ist die Trennung des Gerätes in einfach verwendbare Kammerkomponenten und mehrfach verwendbare Komponenten (Treiber, Steuerung). Bevorzugt werden Kontrollelemente (z.B. Luftdetektoren, Drucksensoren) zur Überwachung der ordnungsgemässen Funktion der Vorrichtung eingesetzt. Das Reservoir wird realisiert durch Infusionsbeutel, -flasche oder bevorzugt durch einen Spritzentreiber bzw. eine unter Druck gesetzte Infusionsspritze. Durch die Pumpvorrichtung gemäss der Erfindung wird dabei die Genauigkeitsanforderung an das Reservoir bzw. den Reservoirentleerungsmechanismus, der nur noch zur Befüllung der Pumpvorrichtung dient, erheblich gesenkt.

### Kurze Beschreibung der Zeichnungen

Im folgenden werden Ausführungsbeispiele der Erfindung anhand der Figuren näher erläutert. Dabei zeigt bzw. zeigen
Figur 1 schematisch die Anordnung der Pumpvorrichtung bei einem Infusionsspritzen-Treiber;
Figur 2 die Anordnung der Pumpvorrichtung bei einer Infusionsflasche oder einem Infusionsbeutel;
Figur 3 die Steuerung mehrerer Pumpsysteme von einem Rechner aus;
Figur 4 grob schematisch die Pumpvorrichtung ohne Antriebselemente in Spülstellung ;
Figur 5 eine Ausführungsform der Pumpvorrichtung ebenfalls in Spülstellung;
Figur 6 die Ausführungsform von Figur 4 in der Stellung zur Befüllung der Pumpenkammer;
Figur 7 die Befüllung der Pumpenkammer mit einer ersten Kolbenstellung;
Figur 8 eine weitere Kolbenstellung;
Figur 9 die Stellung zur Entleerung der Pumpenkammer;
Figur 10 eine Kolbenstellung während der Entleerung;
Figur 11 das Ende der Entleerung der Pumpenkammer;
Figur 12 die Stellung zur erneuten Befüllung;
Figur 13 eine weitere Ausführungsform der Pumpvorrichtung in Befüllstellung der Pumpenkammer;
Figur 14 schematisch eine Pumpvorrichtung mit deren Antriebsteil;
Figur 15 eine Ausführungsform der Pumpvorrichtung mit Dichtungen am Gehäuse und am Küken;
Figur 16 eine Seitenansicht der Pumpvorrichtung von Figur 15; und
Figuren 17-19 Detailansichten der Dichtungen in verschiedenen Stellungen der Pumpvorrichtung.

### Bester Weg zur Ausführung der Erfindung

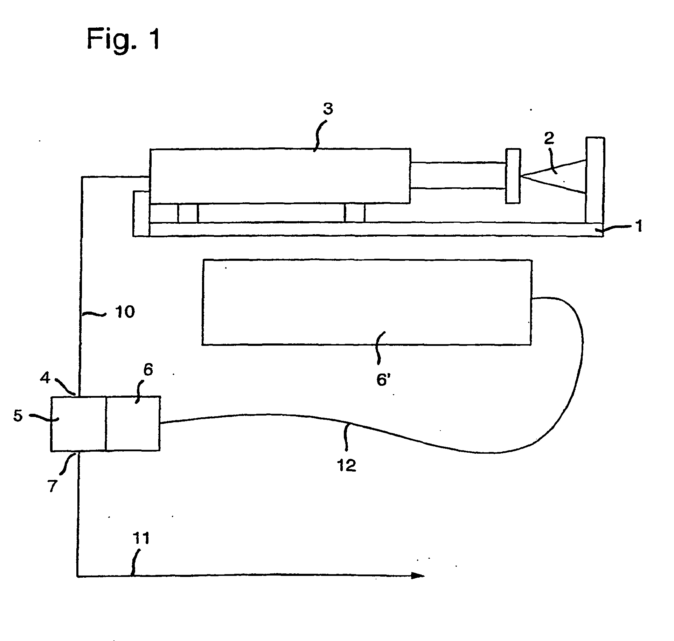

Figur 1 zeigt grobschematisch die bevorzugte Verwendung der medizinischen Pumpvorrichtung bzw. das Pumpsystem. Dieses umfasst einerseits eine an sich bekannten Infusionsspritzen-Treiber mit einem Infusionsspritzen-Schlitten 1, welcher eine Infusionsspritze 3 aufnehmen kann, die von einem Spritzentreiber 2 (im einfachsten Fall einer Feder) betätigt wird, so dass ein Ausstossen der Flüssigkeit in der Infusionsspritze 3 mit einer vorbestimmten Menge pro Zeiteinheit (Motor) oder mit einem relativ konstanten Druck (Feder) erfolgt. Entsprechende Infusionsspritzen-Treiber sind bekannt und werden hier nicht weiter erläutert. Nach Stand der Technik wird der Ausgang der Spritzenpumpe 1 direkt mit dem Patienten gekoppelt, bzw. speist einen Katheter. Gemäss der Erfindung wird nun ein Pumpsystem geschaffen, bei welchem die Infusionsspritze 3 lediglich als Speisung der Pumpvorrichtung 4-7 über die Leitung 10 dient. Die Leitung 10 führt dabei vom Ausgang der Spritzenpumpe 1 zu einem Eingangsanschluss 4 der Pumpvorrichtung 4-7. Am Ausgangsanschluss der Pumpvorrichtung ist wiederum eine Leitung 11 angeschlossen, welche zum Patienten führt. In der dargestellten schematischen Form weist die Pumpvorrichtung die eigentliche Pumpeinheit 5 sowie eine davon trennbar angeordnete Antriebseinheit 6 auf, welche die eigentliche Pumpeinheit antreibt. Sinn dieser bevorzugten Unterteilung ist es, dass die Pumpeinheit 5 jeweils für jeden Einsatz durch eine neue zum einmaligen Gebrauch bestimmte Pumpeinheit ausgewechselt werden kann und die Antriebseinrichtung 6 beibehalten werden kann. Im gezeigten Beispiel wird die Antriebseinheit 6 von einer nicht näher dargestellten Steuer- und Anzeigeeinheit 6' betrieben, welche über eine Steuerleitung 12 die Antriebseinheit steuert. An der Steuereinheit 6' kann die gewünschte Fördermenge der Pumpvorrichtung 4-7 eingestellt und angezeigt werden. Diese dargestellte Ausbildung mit getrennten Einheiten ist natürlich nur als Beispiel zu verstehen, und es könnte die Pumpvorrichtung auch als nur eine Einheit vorgesehen sein, die die Teile 4, 5, 6 und 6' vereinigt. Das in Figur 1 gezeigte Pumpsystem weist die Nachteile der bekannten Spritzenpumpen, welche in den Untersuchungen gemäss Literaturverzeichnis dargestellt sind, nicht auf, da die Pumpvorrichtung 4-7 die Probleme mit der Nachgiebigkeit, bzw. der Empfindlichkeit auf hydrostatische Druckänderungen und mit anderen Unzulänglichkeiten der Anordnung aus Spritze und deren Betätigungseinrichtung vermeiden kann. Die Pumpvorrichtung 4-7 des Systems sorgt dafür, dass im wesentlichen unabhängig von Eingangsdruckschwankungen immer dasselbe Volumen pro Zeiteinheit gefördert wird.

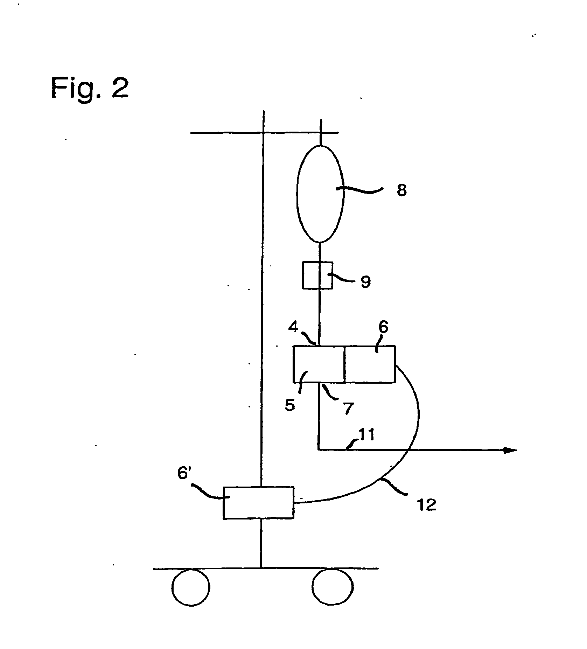

Ähnliche Vorteile ergeben sich, wenn wie in Figur 2 gezeigt, die medizinische Pumpvorrichtung 4-7 an den Ausgang eines herkömmlichen Infusionsbehälters 8, dem eine Tropfenkammer 9 nachgeschaltet sein kann, angeschlossen wird. Auch diesfalls führt die Leitung 10 zum Eingangsanschluss 4 der Pumpvorrichtung 4-7 und die Leitung 11 führt vom Ausgangsanschluss 7 der Pumpvorrichtung zum Patienten. Nachfolgend werden anhand mehr oder weniger schematischer Darstellungen verschiedene Pumpvorrichtungen erläutert, wobei in der Regel nur die eigentliche Pumpeinheit 5 dargestellt wird und die Antriebseinheit 6 sowie die Steuer- und Anzeigeeinheit 6' nicht dargestellt werden. Die Ausgestaltung der jeweiligen Antriebseinheit 6 ist durch die Bewegung des Kolbens und des drehbaren Elementes in der Pumpeinheit 5, das noch beschrieben wird, vorgegeben und damit für den Fachmann auf vielfältige Weise ohne weitere Anleitung im Rahmen seines Fachwissens zu realisieren. Es können dabei Elektromotoren als Linearmotoren, Schrittmotoren oder normale Motoren mit Getrieben zur Anwendung kommen. Auch pneumatische oder hydraulische Antriebe sind möglich. Die jeweilige Steuer- und Anzeigeeinheit 6' wird ebenfalls in für den Fachmann grundsätzlich bekannter Weise in elektronischer, vorzugsweise mikroprozessorgesteuerter Form ausgeführt und muss hier nicht näher erläutert werden. Da gemäss der Erfindung das Pumpvolumen jedes Pumpvorganges als konstant angesehen werden kann, ist insbesondere eine einfache Festlegung und Kontrolle der Flussrate durch Vorgabe und Zählung der Pumpenhübe möglich. In den nachfolgenden Ausführungsbeispielen werden grundsätzlich gleiche Elemente wie in den Figuren 1 und 2 mit denselben Bezugszeichen wie in diesen Figuren bezeichnet.

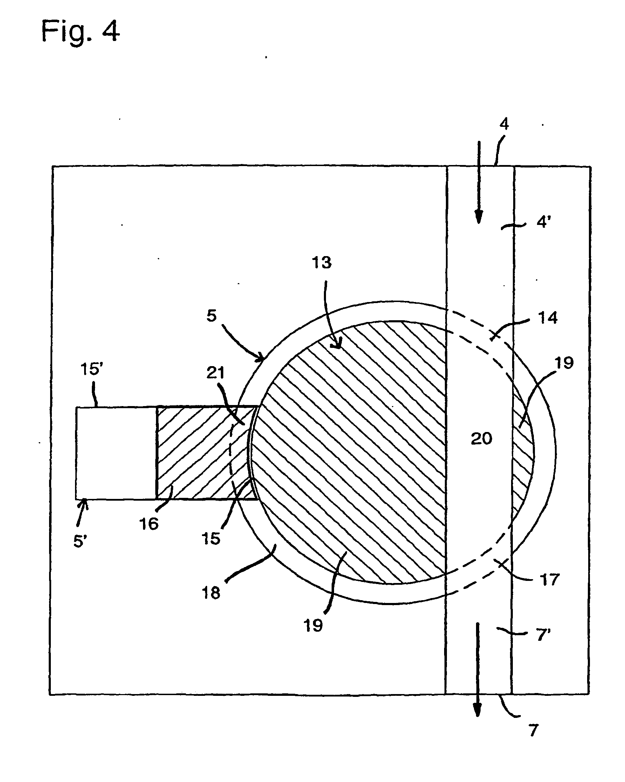

Figur 4 zeigt in einer teilweise horizontal geschnittenen schematischen Darstellung die Pumpvorrichtung in Draufsicht mit abgenommenem Deckel, welche Pumpvorrichtung durch die Umrandung generell dargestellt ist, die aber in den weiteren Figuren zur Vereinfachung nicht verwendet wird. Die Vorrichtung weist einen Eingangsanschluss 4 auf, an welchen die bereits erwähnte Leitung 10 von der Spritzenpumpe oder von einem Infusionsgefäss anschliessbar ist. Die entsprechenden Anschlussmittel sind nicht dargestellt; dabei kann es sich um beliebige bekannte Anschlussmittel handeln. Die Vorrichtung besitzt weiter einen Ausgangsanschluss 7, an welchen die erwähnte Leitung 11 auf dieselbe Weise anschliessbar ist. Die Pumpvorrichtung hat dabei Anschlüsse für die gezeigten Infusionsleitungen (distal und proximal) oder hat selber kurze Leitungen mit Anschlüssen für die Infusionsleitungen (distal und proximal) oder weist bereits angeschlossene, z.B. angeschweisste Infusionsleitungen auf, z.B. auch die proximale Leitung mit einer Tropfkammer beim Beutelsystem, oder es ist eine Kombination dieser Anschlussvarianten vorgesehen. Weiter umfasst die Vorrichtung die eigentliche Pumpeinheit 5, sowie die in der Figur 4 nicht dargestellte Antriebseinheit und Steuereinheit. Die Pumpvorrichtung weist nun ein durch Drehbewegung umschaltbares Mehrwegventil 13 auf, welches in einem zylindrischen Ventilgehäuse 18 ein drehbares, zylindrisches Ventilelement 19 aufweist, das einen Kanal 20 beinhaltet. Der Kanal 20 erstreckt sich dabei im Element 19 bei der gezeigten Stellung desselben von der Öffnung 14 im Gehäuse 18 zur Öffnung 17 im Gehäuse 18, wobei der Kanal mit z.B. zylindrischem oder rechteckigem Querschnitt mit den entsprechenden Öffnungen im Gehäuse 18 fluchtet, so dass sich Flüssigkeit vom Eingangsanschluss 4 durch ein eventuell nur sehr kurzes oder gar nicht vorhandenes Leitungsstück 4' durch die Gehäuseöffnung 14, den Kanal 20 und durch die Gehäuseöffnung 17 und durch das eventuell nur sehr kurze oder nicht vorhandene Leitungsstück 7' zum Ausgangsanschluss 7 der Pumpvorrichtung fliessen kann, wenn sich das Ventilelement 19 in der gezeigten Stellung befindet. Die Zeichnung ist dabei vereinfacht gehalten, so dass die Leitungsstücke 4' und 7' mit dem Kanal 20 in der Figur eine Einheit bilden, was natürlich tatsächlich nicht der Fall ist, da diese Leitungsabschnitte 4' und 7' und der Kanal 20 getrennte Abschnitte sind, die in der gezeigten Stellung des Drehelementes 19 fluchten. Mit 15' ist die Wandung einer Kammer bezeichnet, in welcher ein starrer, aber verschiebbarer Kolben 16 angeordnet ist. Diese Teile bilden den eigentlichen Pumpenteil 5'. Die Kammer ist vermittels einer Gehäuseöffnung 21 im Gehäuse 18 zum Element 19 hin offen, wobei in der gezeigten Stellung die Kammer weder mit dem Eingangsanschluss 4 noch mit dem Ausgangsanschluss 7 in Verbindung steht und der Kolben 16 in seiner vorderen Endposition ist. Die Kammer 15 zwischen Kolbenstirnseite und Gehäuseöffnung 21 hat bei dieser Kolbenstellung praktisch kein Volumen. Einlass, Auslass und die Kammer bzw. die Öffnungen 14, 17 und 21 sind gleichmässig um das Drehventilgehäuse verteilt, somit in einem Winkelabstand von jeweils 120°. Der Kolben weist bevorzugt die gezeigte, hohl gewölbte Stirnseite bei der Gehäuseöffnung 21 auf, welche Stirnseite an die zylindrische Aussenkontur des Elementes 19 angepasst ist.

In der gezeigten Stellung der Pumpvorrichtung ist ein Flüssigkeitsfluss von der Spritzenpumpe bzw. dem Infusionsbehälter durch die Vorrichtung hindurch möglich, was das Durchspülen der Vorrichtung erlaubt. Der geradlinig verlaufende Kanal 20 weist dabei keine Kanten und Vorsprünge auf, an denen sich Luftblasen anlagern können. Bevorzugt ist auch, wenn der Kanal mit dem Einlass 4 bzw. Auslass 7 so fluchtet, dass sich keine Kanten und Vorsprünge bilden.

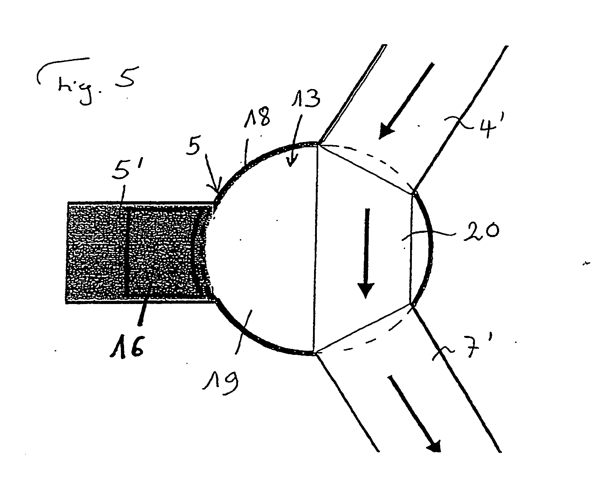

Figur 5 zeigt eine abgewandelte Ausführungsform, bei welcher wiederum gleiche Bezugszeichen wie in Figur 4 grundsätzlich gleiche Elemente bezeichnen. In diesem Fall sind die Leitungsabschnitte 4' und 7' zum Kanalverlauf 20 hin abgewinkelt angeordnet. Ansonsten bleibt die Pumpvorrichtung grundsätzlich dieselbe. Der Pumpenteil ist in Figur 5 nur generell mit der Bezugsziffer 5' bezeichnet, ohne dass darin Kolben und Kammer genauer dargestellt wären. Auch die Gehäuseöffnungen 14 und 17 sind nur angedeutet. Die Leitungsabschnitte 4' und 7' sowohl in Figur 4 als auch in Figur 5 können auch ganz entfallen, wenn der Eingangsabschluss 4 bzw. der Ausgangsanschluss 7 direkt am Gehäuse 18 des Drehventils 12 ausgebildet ist. Allenfalls sind dann die Leitungsabschnitte 4', 7' nur sehr kurze Leitungsstummel. Die in den Figuren 4 und 5 gezeigte Bypass-Stellung, durch welche die Pumpvorrichtung bzw. das Pumpsystem mit Infusionslösung durchspült werden kann, dient, wie gesagt, zum Ausspülen oder zum Aufsteigenlassen von Luftblasen. Letzteres ist besonders relevant, wenn oberhalb der Pumpvorrichtung eine Tropfkammer verwendet wird, wie dies bei der Verwendung eines Infusionsbehälters üblich ist. Eine Rüttelfunktion (feine Vibrationen) der Pumpvorrichtung in der Bypass-Stellung kann das Aufsteigen von Luftblasen fördern.

Die Bypass-Stellung wird im eigentlichen Pumpbetrieb, der nachfolgend erläutert wird, nicht mehr eingenommen, so dass dabei keine direkte Verbindung zwischen Einlass und Auslass besteht.

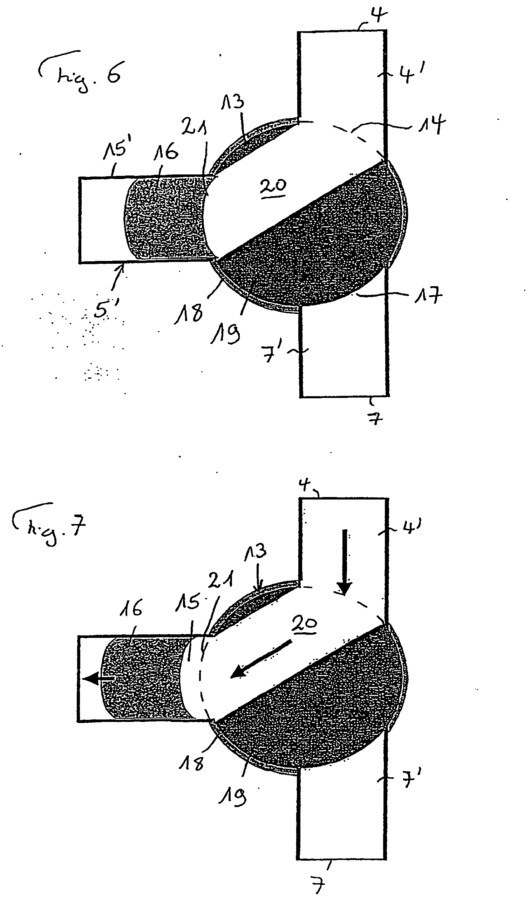

Figur 6 zeigt nun die Aspirationsstellung der Pumpvorrichtung gemäss Figur 4, bei welcher das Element 19 des Drehventils so gedreht worden ist, dass der Kanal 20 den Eingangsanschluss 4 mit dem Pumpenteil 5' verbindet. Der Kanal 20 kommt dabei so zu liegen, dass er die Gehäuseöffnung 14 mit der Gehäuseöffnung 21 des Ventilgehäuses 18 verbindet und somit Flüssigkeitseintritt in die Kammer des Pumpenteils 5' erlaubt. Figur 7 zeigt eine resultierende Stellung, bei welcher der Flüssigkeitsfluss in die Kammer hinein erfolgt, wobei der Kolben 16 durch den Flüssigkeitsdruck zurückgestossen wird oder bevorzugt durch ein Antriebsmittel der nicht dargestellten Antriebseinheit 6 zurückbewegt wird. In dieser Stellung des Elements 19 ist die Leitung 7' bzw. der Ausgangsanschluss 7 blockiert und erhält keine Flüssigkeit zugeführt. Diese Stellung kann daher auch als Blockierungsstellung (Okklusionsstellung) der Pumpvorrichtung verwendet werden, wenn der Flüssigkeitsfluss durch entsprechende Einstellung der Pumpvorrichtung blockiert werden soll. Figur 8 zeigt die abgeschlossene Befüllung der Kammer der Pumpe, bei welcher sich der Kolben 16 in seiner hinteren Totpunktlage befindet. Die in der Kammer befindliche Flüssigkeitsmenge bzw. die ausstossbare Flüssigkeitsmenge liegt dabei z.B. im Bereich von 0,001 ml bis 0,01 ml, was einem Volumen von 1 mm3 bis 10 mm3 entspricht, wobei die ausstossbare Menge durch den Querschnitt des Raumes, in dem die Kammer gebildet ist und dem Kolbenhub bestimmt ist, der die Kammerlänge definiert. Der angegebene Bereich ist ein bevorzugter Bereich für das Pumpvolumen der vorliegenden medizinischen Pumpvorrichtung und gilt auch als bevorzugter Bereich für die weiteren in der Beschreibung geschilderten Varianten. Auch bei der Aspirationsstellung kann ein feines Vibrieren (z.B. mittels der Antriebseinheit 6, bewirkt werden, um Luftblasen (microairbubbles) nach oben steigen zu lassen.

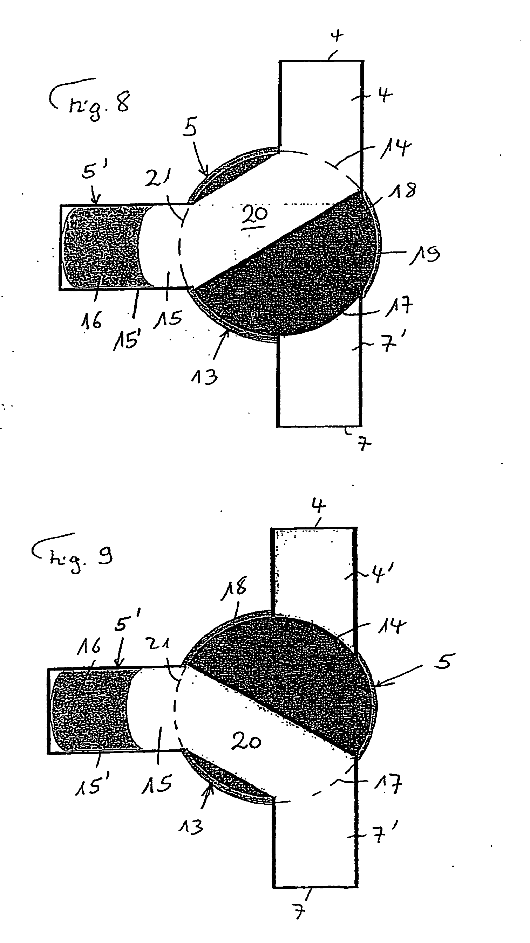

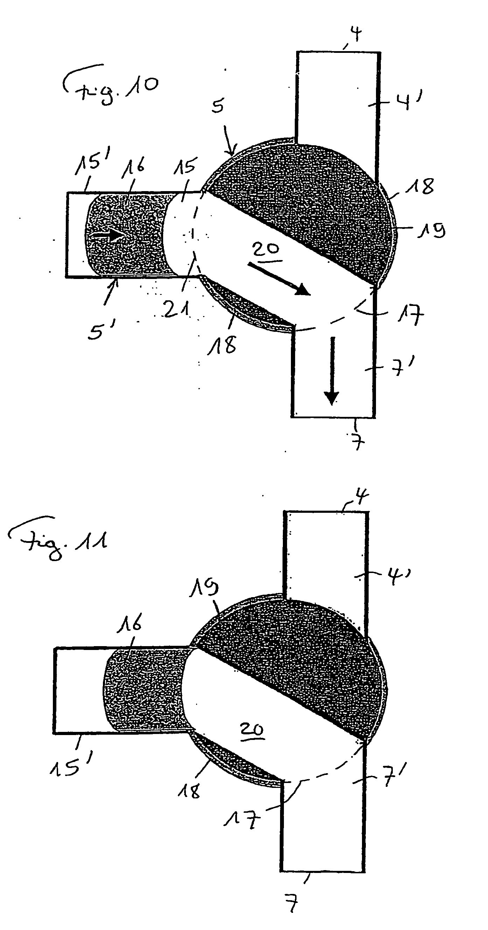

Nachdem somit die Kammer befüllt worden ist, wird im nicht-blockierenden Betrieb der Pumpvorrichtung das drehbare Ventilelement 19 weitergedreht, so dass der Kanal 20 die Öffnung 21 der Kammer mit der Öffnung 17 verbindet, wie dies in Figur 9 dargestellt ist. In dieser Ausstossstellung bleibt der weitere Eintritt von Flüssigkeit durch den Eingangsanschluss 4 und den Leitungsstummel 4' durch das Element 19 blockiert. Die Figuren 10 und 11 zeigen nun den eigentlichen Pumpvorgang, bei welchem der Kolben 16 zur Öffnung 21 des Ventilgehäuses 18 hin durch die nicht dargestellte Antriebseinheit bewegt wird, wodurch die in der Kammer befindliche Flüssigkeit aus dieser ausgestossen und durch den Ausgangsanschluss 7 der Pumpvorrichtung abgegeben wird. Figur 11 zeigt die entsprechende Endstellung, bei welcher das gesamte Kammervolumen an Flüssigkeit ausgestossen worden ist. Auch diese Stellung kann als Blockierstellung der Pumpvorrichtung (Okklusionsstellung) verwendet werden, wenn die Pumpe durch die Bedienungsperson entsprechend eingestellt worden ist, dass keine Flüssigkeit gepumpt werden soll.

Dahingegen wird im eigentlichen Pumpbetrieb nachfolgend der Stellung von Figur 11 durch Drehung des Ventilelementes 19 durch die nicht dargestellte Antriebseinheit und die nicht dargestellte Steuereinheit, so dass eine erneute Befüllung der Pumpkammer von der Eingangsseite (Eingangsanschluss 4) der Pumpvorrichtung her erfolgen kann. Nach vollständiger Befüllung der Kammer wird wiederum die Position der Figuren 9 bis 11 durchlaufen, bei dem ein Ausstossen erfolgt, wonach wiederum die Befüllungsposition entsprechend der Figur 12 bzw. der Figuren 6 und 7 angenommen wird. Auf diese Weise ergibt sich ein ständiges Befüllen und Entleeren der Pumpenkammer der Pumpeinheit 5, wodurch die Pumpvorrichtung auf gewünschte Weise die geringen Flüssigkeitsmengen fördert. Am Ende der gewünschten Betriebszeit kann dann wieder eine der Stellungen als Okklusionsstellung beibehalten werden.

Das Vorsehen des Drehventils 13 mit dem geradlinigen Kanal 20, ergibt dabei eine sehr einfache Konstruktion, die auch entsprechend durch eine einfache Antriebseinheit antreibbar ist. Entgegen an sich bekannten Dreiweghähnen, bei welchen T-förmige oder L-förmige Kanäle in den Küken vorgesehen sind, wird bei dem dargestellten Ventil 13 nur ein Kanal verwendet, welcher entweder das Spülen erlaubt, wie in den Figuren 4 und 5 gezeigt und danach durch die abwechselnden anderen beiden Stellungen das Befüllen und Entleeren der Pumpenkammer erlaubt oder in einer dieser beiden Stellungen bei abgeschalteter Antriebseinheit den Durchfluss durch die Pumpvorrichtung sperrt. T- oder L-förmige Dreiweghähne würden es nicht erlauben eine direkte Bypass-Verbindung zu schaffen und würden das Risiko der Bildung von Luftansammlungen aufweisen.

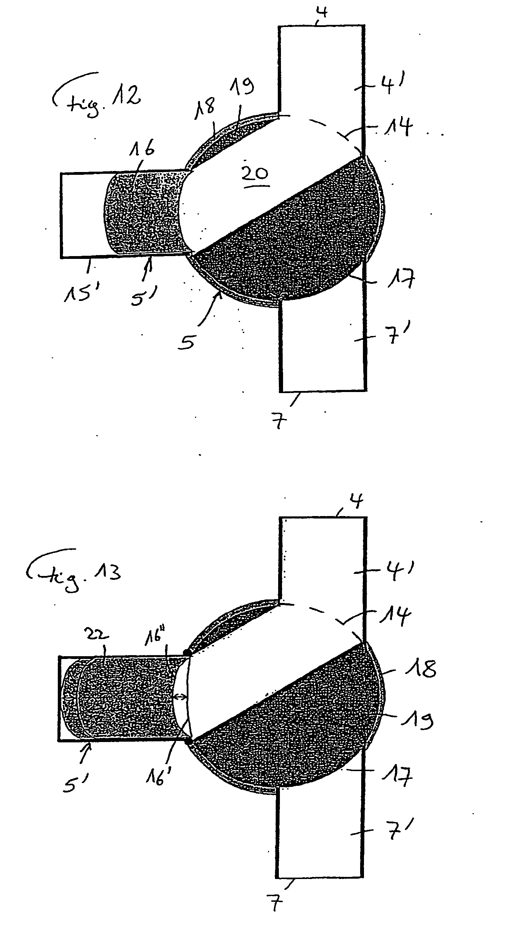

Figur 13 zeigt eine Variante mit dem grundsätzlich gleichen Drehventil 13, bei welcher indes das Pumpenteil 5' anstelle eines Kolbens einen elastischen oder quasi-elastischen Körper, z.B. eine Membran-16' aufweist, die durch den Flüssigkeitsdruck verformt werden kann, so dass ein bestimmtes Kammervolumen gefüllt wird. In der Figur 13 ist dabei die Membran einmal in ihrer Ruhestellung mit 16' bezeichnet und in ihrer teilweise oder ganz befüllten Stellung mit 16". Hinter der Membran kann z.B. eine Flüssigkeit 22 angeordnet sein, welche nur eine bestimmte Verformung der Membran erlaubt. Entsprechende Anordnungen sind aus der CH-Patentanmeldung Nr. 2110/01 bekannt, deren entsprechender Inhalt hierin durch Verweis vollumfänglich aufgenommen ist. Die Membran 16' ist vorzugsweise rundum an dem Umfang der Gehäuseöffnung 21 befestigt, so dass sich ein gleichmässiger Raum für die aufzunehmende Flüssigkeit ergibt, und auf Dichtungen und Gleiträume für den Kolbenzylinder verzichtet werden kann.

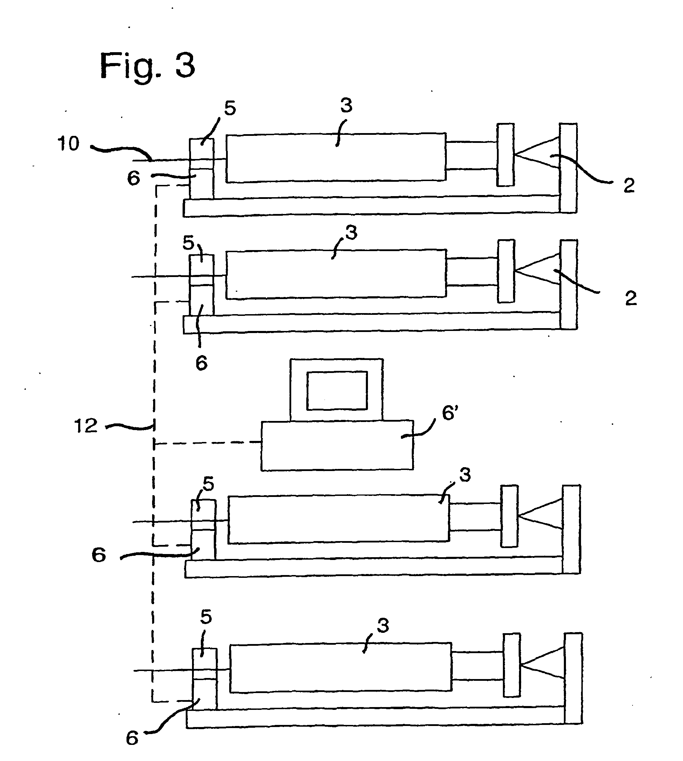

Figur 14 zeigt schematisch noch einmal die Pumpvorrichtung mit Abschnitten der Eingangs- und Ausgangsleitungen und mit dem Pumpenteil 5' einmal ohne Antriebseinheit und einmal mit einer angesetzten Antriebseinheit 6, welche über die Leitung 12 mit Energie und Signalen versorgt wird, welche die entsprechende Bewegung der Antriebseinheit zur Steuerung des Pumpenteils und des Drehventils liefert. Diese Antriebseinheit bzw. der Pumpentreiber kann dabei durch Befestigungsmittel einfach an der Pumpeinheit und dem Drehventil angesetzt werden. Sie bewegt die entsprechenden Elemente z.B. mittels zweier Schrittmotoren und kann z.B. einen Luftblasendetektor 24 für den Eingangsanschluss aufweisen und vorteilhafterweise eine Drucksensoreinheit 24' im Ausgangsanschluss, um distale Okklusionen der Leitung 11 zu detektieren. Ebenfalls ist am zuführenden Anschluss eine Drucksensoreinheit möglich, welche z.B. eine leere Infusionsquelle frühzeitig melden könnte (bevor Luft angesaugt wird oder eine Okklusion in der zuführenden Leitung melden könnte). Die beschriebene Pumpvorrichtung wird wie in den Figuren 1 bis 3 gezeigt mit einem Infusionsspritzen-Treiber oder mit einem Infusionsbehälter kombiniert. Figur 1 zeigt dabei schematisch die erfindungsgemässe Pumpvorrichtung mit einem Infusionsspritzen-Treiber mit entsprechenden Infusionsleitungen. Die Vorrichtung ist in der Infusionsleitung eingebaut und wird an die gefüllte Infusionsspritze angeschlossen (für die Anschlussmöglichkeiten wird auf die bereits erwähnten verschiedenen Möglichkeiten verwiesen). Die Infusionsspritze ist in diesem Beispiel auf einem Halter bzw. Schlitten mit Druckfeder eingespannt, welche einen dosierten regelmässigen Druck auf die Infusionsspritze ausübt, um die Pumpvorrichtung bei der Füllung zu unterstützen und um elektrische Energie zu sparen. Die Figur 1 zeigt die Pumpvorrichtung mit der Antriebseinheit. Figur 2 zeigt die Pumpvorrichtung anhand eines Infusionsbeutel/Flaschen-Systems mit Infusionsleitungen, wobei eine Tropfkammer vorgesehen ist, die der Luftabsonderung dient. Die Pumpvorrichtung ist in die Infusionsleitung eingebaut, die in die Infusionsflasche/Beutel eingesteckt ist, wobei die Tropfkammer wie üblich durch manuelles Quetschen gefüllt wird, währenddem die Pumpe in Okklusionsstellung ist, womit die Infusionsleitung gegen unten verschlossen ist, damit keine Luft von unten angesaugt wird. Anschliessend wird die Pumpvorrichtung durch die Bedienungsperson bzw. Betätigung der Steuereinheit 6' und entsprechend der Antriebseinheit 6 in die Spülstellung gebracht, so dass ein freier Fluss durch die Pumpe möglich ist und die Infusionsleitung mit Infusionslösung durchgespült und von Luft gereinigt wird. Danach wird der Pumpbetrieb ausgelöst. Alternativ kann die Pumpvorrichtung auch manuell in Durchflussstellung gebracht werden, solange die Antriebseinheit noch nicht an die Pumpvorrichtung angesetzt ist. Dies erfolgt in diesem Fall nach der Spülung. Figur 3 zeigt schematisch mehrere Pumpsysteme mit Infusionsspritzen-Treibern und Pumpeinheiten/Antriebseinheiten 5/6, welche jeweils nicht von einer einzelnen Steuereinheit 6' gesteuert werden, sondern von einer zentralen Steuereinheit, z.B. einem PC, mittels mehrerer Steuerkabel 12 versorgt, gesteuert und überwacht werden. Die Pumpvorrichtung gemäss der Erfindung weist vorzugsweise eine Anzeigeeinrichtung auf, z.B. an der Antriebseinheit 6 und/oder der Steuereinheit 6'. An der Anzeigeeinrichtung sollte das Medikament und/oder die Flussrate erkennbar sein. Bei einer Steuerung durch einen Computer (Figur 3) sind diese Angaben vorzugsweise zusätzlich (oder auch nur) auf dem Monitor des Computers dargestellt.

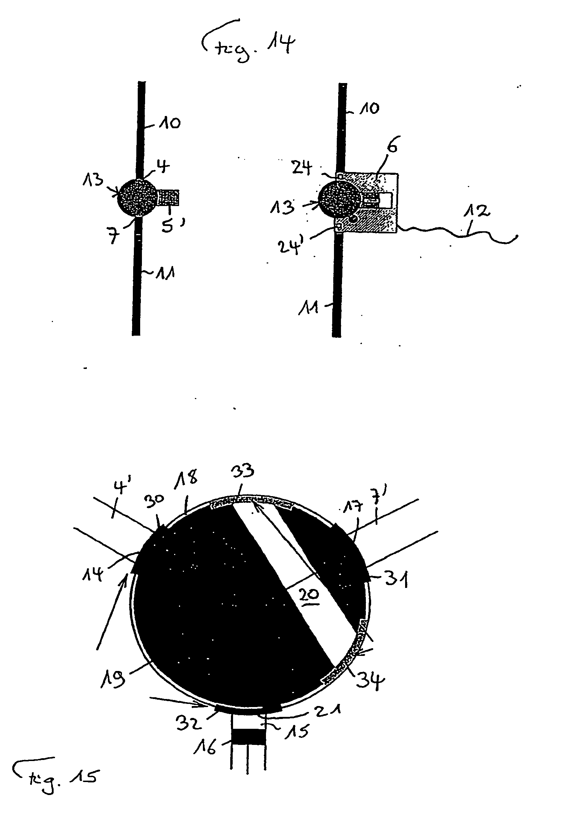

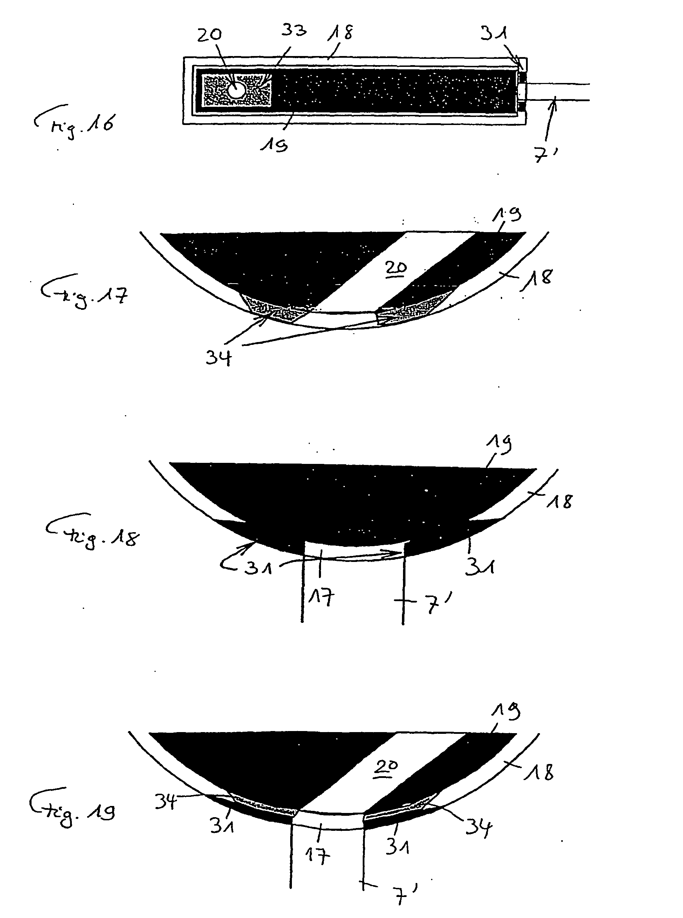

Figur 15 zeigt eine weitere schematische teilweise geschnittene Ansicht der Pumpvorrichtung, wobei anhand dieser verschiedene Dichtungen erläutert werden, welche einerseits feststehende, mit dem Ventilgehäuse 18 verbundene Dichtungen 30, 31 und 32 umfassen, die bei den jeweiligen Öffnungen im Ventilgehäuse angeordnet sind, sowie am jeweiligen Ende des Kanals 20 des Elements 19 angeordnete Dichtungen 33 und 34, welche sich mit dem Element 19 zusammen bewegen. Figur 16 zeigt eine entsprechende Seitenansicht, bei welcher sowohl eine der beweglichen Dichtungen als auch eine feststehende Dichtung im Ventilgehäuse dargestellt ist. Die Figuren 17 und 18 zeigen Detailansichten der entsprechenden Dichtungen, wobei deren Abschrägung und Abrundung dargestellt ist, welche ein möglichst reibungsarmes Gleiten des Elements 19 im Ventilgehäuse 18 ermöglichen. Figur 19 schliesslich zeigt die Stellung, bei welcher der Kanal 20 des Elements 19 mit einer der Gehäuseöffnungen des Pumpengehäuses fluchtet, wobei die entsprechenden Dichtungen übereinander zu liegen kommen und sich entsprechend verformen. Demgemäss werden elastische bekannte Dichtungsmaterialien verwendet, welche resistent gegen die Infusionslösung sind. Da der dargestellte Ventilteil der Pumpvorrichtung in der Regel als Wegwerfteil ausgestaltet ist, an welchen die Antriebseinheit 6 angedockt wird, bietet dabei die Langzeitlebensdauer der Dichtungen keine Probleme.

### Literatur

*1) Cook RI. Syringe pump assemblies and the natural history of clinical technology (2000) CAn J Anesth 47: 929-935.*
*2) Lönnqvist PA (2000) How continuous are continuous drug infusions? Intensive Car Medicine 26:660-1.*
*3) Krauskopf KH, Rascher J*, *Brandt L (1996) Störung der kontinuierlichen, pumpengesteuerten Applikation kardiovaskulär wirksamer Pharma durch den hydrostatischen Druck. Anaesthesist 45: 449-452.*
*4) Lönnqvist PA, Löfqvist B (1997) Design flaw can convert commercially available continuous syringe pumps to intermittent bolus injectors. Intensive Care Med 23: 9989-1001.*
*5) Weiss M, Baenziger O*, *Neff T, Fanconi S (2000) Influence oif infusion line compliance on drug delivery during acute line loop formation. Intensive Care Medicine 26: 776-779.*
*6) Weiss M, Fischer J, Neff T, Baenziger O (2000) Syringe plunger design affects drug delivery from syringe infusion pumps. Anaesthesia 55*: *1094-1098.*
*7) Neff T, Fischer J*, *Schulz G*, *Baenziger O, Weiss M (2001) Infusion pump performance with vertical displacement*: *effect of syringe pump and assembly type. Intensive Care Medicine 27*: *287-291.*
*8) Weiss M, Hug MI, Neff T, Fischer J (2000) Syringe size and flow rate affect drug delivery from syringe pumps. Canadian Journal of Anesthesia 47*: *1031-1035.*
*9) Weiss M, Fischer J, Schulz G, Neff T, Baenziger O (2000) Do antisiphon valves reduce flow irregularities* of *syringe pumps? Anaesthesia and Intensive Care 28: 680-683.*
*10) Kim DW, Steward DJ (1999) The effect of syringe size on the performance of an infusion pump. Paediatric Anaesthesia 9*: *335-7.*
*11) Weiss M, Neff T, Fischer J, Gerber AC (2000) The effect of infusion line compliance on syringe pump performance. Paediatric Anaesthesia 10: 595-599.*
*12) Schulz G, Fischer J, Neff T, Baenziger O, Weiss M (2000) Der Einfluss von Lufteinschluss in der Infusionsspritze auf die Funktion von Spritzenpumpen. Anaesthesist 49: 1018-1023.*
*13) Neff T, Fischer J, Fehr S, Baenziger O*, *Weiss M (2001) Start-up delays of syringe infusion pumps. Paediatric Anaesthesia, in press.*
*14) Neff T, Fischer J, Fehr S, Baenziger 0, Weiss M (2001) Evaluation of the FASTSTART mode for reducing start-up delay in syringe pump infusion systems. Swiss Medical Weekly; in press.*
*15) McCarroll C, McAtamney D, Taylor R (2001) Altera tion in flow delivery with antisyphon devices. Anaesthesia 55: 355-357.*
*16) Capes DF, Dunster KR, Sunderland VB et al. Fluctuations in syringe pump infusion systems: Association with blood pressure variations in infants. Am J Health-Syst Pharm 1995; 52: 1 646-1653.*

## Patentansprüche

1. Medizinische Pumpvorrichtung (4-7) mit einem Eingangsanschluss (4) für eine erste Fluidleitung (10) und einem Ausgangsanschluss (7) für eine zweite Fluidleitung (11) sowie einem zwischen Eingangsanschluss und Ausgangsanschluss liegenden Pumpenteil, welches mindestens eine steuerbar über den Eingangsanschluss befüllbare und über den Ausgangsanschluss entleerbare Kammer (15) aufweist, wobei die Pumpvorrichtung zur Herstellung der Verbindung zwischen Eingangsanschluss (4) und Ausgangsanschluss (7) oder Kammer (15) und zwischen Kammer (15) und Ausgangsanschluss (7) eine Drehventilanordnung (13) aufweist, wobei diese um das Drehventilgehäuse (18) verteilte Öffnungen (14, 17, 21) derselben aufweist, die mit dem Eingangsanschluss, dem Ausgangsanschluss und der Kammeröffnung korrespondieren und wobei das Drehelement (19) des Ventils einen Kanal (20) zur wahlweisen Verbindung von Eingangsanschluss, Ausgangsanschluss und Kammer aufweist, **dadurch gekennzeichnet, dass** die Anschlüsse und die Kammer bzw. die Öffnungen gleichmässig im Drehventilgehäuse angeordnet sind, derart, dass die Mitten der drei Öffnungen jeweils um 120 Grad versetzt angeordnet sind, und dass der Kanal ein gerade verlaufender Kanal (20) ist.

2. Medizinische Pumpvorrichtung nach Anspruch 1, **dadurch gekennzeichnet, dass** die Kammer (15) allseits starre Kammerwände aufweist, von denen mindestens eine beweglich angeordnet ist.

3. Medizinische Pumpvorrichtung nach Anspruch 1, **dadurch gekennzeichnet, dass** nur ein Kanal (20) vorgesehen ist.

4. Medizinische Pumpvorrichtung nach einem der Ansprüche 2 oder 3, **dadurch gekennzeichnet, dass** die starre aber bewegliche Kammerwand von mindestens einem starren Kolben (16) oder Stössel gebildet wird.

5. Medizinische Pumpvorrichtung nach einem der Ansprüche 2 oder 3, **dadurch gekennzeichnet, dass** die starre aber bewegliche Kammerwand von einer elastischen oder quasi-starren Membran (16') gebildet wird, welche als starre Kammerwand wirkt, insbesondere indem sie vollständig von einem nichtkompressiblen Beaufschlagungsmittel, insbesondere einer Flüssigkeit (22), beaufschlagt wird und gegebenenfalls mit einem Auslenkungsbeschränkungsmittel versehen ist.

6. Medizinische Pumpvorrichtung nach einem der Ansprüche 1 bis 5, **dadurch gekennzeichnet, dass** deren Antrieb im wesentlichen in einem von der Vorrichtung lösbaren Antriebsteil (6) angeordnet ist.

7. Medizinische Pumpvorrichtung nach einem der Ansprüche 1 bis 6, **dadurch gekennzeichnet, dass** elastische Dichtungselemente (30-34) sowohl im Bereich des Kanalendes des Drehelementes (19) des Ventils (13) an diesem als auch im Bereich der Gehäuseöffnungen (14, 17, 21) am Gehäuse (18) vorgesehen sind.

8. Medizinisches Pumpsystem umfassend einen Infusionsspritzen-Treiber (1) oder einen Infusionsbehälter (8, 9) sowie eine mit deren bzw. dessen Ausgang verbundene Pumpvorrichtung nach einem der Ansprüche 1 bis 7.

## Claims

1. A medical pump device (4-7)with an inlet connector (4) for a first fluid conduit (10) and an outlet connector (7) for a second fluid conduit (11) as well as a pump part located between the inlet connector and the outlet connector which comprises at least one chamber (15) which is in a controlled manner fillable by the inlet connector and drainable through the outlet connector, wherein the pump device comprises a rotary valve arrangement (13) for establishing the communication between the inlet connector (4) and the outlet connector (7) or the chamber (15) and between the chamber (15) and the outlet connector (7), whereby it comprises openings (14, 17, 21) distributed around the rotary valve housing (18) which correspond to the inlet connector, the outlet connector and the chamber opening, and whereby the rotary element (19) of the valve includes a channel (20) for a selectable communication between the inlet connector, the outlet connector and the chamber, **characterized in that** the connectors and the chamber and openings, respectively, are arranged uniformly in the rotary valve housing, so that the centers of three openings are respectively arranged staggered by 120 degrees and that the channel is a rectilinear extending channel (20).

2. A medical pump device according to claim 1, **characterized in that** said chamber (15) includes at all sides rigid chamber walls of which at least one is arranged movably.

3. The medical pump device according to claim 1, **characterized in that** only one channel (20) is provided.

4. The medical pump device according to one of claims 2 or 3, **characterized in that** the rigid but moveable chamber wall is formed by at least one rigid piston (16) or plunger.

5. The medical pump device according to one of claims 2 or 3, **characterized in that** the rigid but moveable chamber wall is formed by a elastic or quasi-rigid membrane (16') which acts as a rigid chamber wall, in particular **in that** it is completely acted upon by a non-compressible acting medium, in particular a liquid (22), and possibly is equipped with deflection limiting means.

6. The medical pump device according to one of claims 1 to 5, **characterized in that** its drive is arranged substantially in a drive part (6) detachable from the device.

7. The medical pump device according to one of claims 1 to 6, **characterized in that** elastic sealing elements (30-34) are provided in the area of the channel end of the rotary element (19) of the valve on same, as well as also in the area of the housing openings (14, 17, 21) at the housing (18).

8. A medical pump system including an infusion syringe driver (1) or an infusion container (8,9) as well as a pump device according to one of claims 1 to 7 connected to its outlet.

## Revendications

1. Dispositif (4 à 7) médical de pompage, comprenant un raccord (4) d'entrée pour un premier conduit (10) de fluide et un raccord (7) de sortie pour un deuxième conduit (11) de fluide, ainsi qu'une partie de pompe se trouvant entre le raccord d'entrée et le raccord de sortie et ayant au moins une chambre (15) pouvant, de manière commandée, être remplie par le raccord d'entrée et être vidée par le raccord de sortie, le dispositif de pompage, ayant pour ménager la communication entre le raccord (4) d'entrée et le raccord (7) de sortie ou la chambre (15) et entre la chambre (15) et le raccord (7) de sortie, un dispositif (13) de vanne rotative, celui-ci ayant des ouvertures (14, 17, 21) qui sont réparties autour du corps (18) de vanne rotative et qui correspondent au raccord d'entrée et au raccord de sortie et à l'ouverture de chambre et l'élément (19) tournant de la vanne ayant un canal (20) pour faire communiquer au choix le raccord d'entrée et le raccord de sortie et la chambre, **caractérisé en ce que** les raccords et la chambre ou les ouvertures sont disposés uniformément dans le corps de la vanne rotative de façon à ce que les milieux des trois ouvertures soient décalées respectivement de 120° et **en ce que** le canal est un canal (20) rectiligne.

2. Dispositif médical de pompage suivant la revendication 1, **caractérisé en ce que** la chambre (15) a des parois de chambre rigides de tout côté, dont l'une au moins est mobile.

3. Dispositif médical de pompage suivant la revendication 1, **caractérisé en ce qu'**il n'est prévu qu'un canal (20).

4. Dispositif médical de pompage suivant l'une des revendications 2 ou 3, **caractérisé en ce que** la paroi de chambre rigide mais mobile est formée par au moins un piston (16) rigide ou un poussoir.

5. Dispositif médical de pompage suivant l'une des revendications 2 ou 3, **caractérisé en ce que** la paroi de chambre rigide mais mobile est formée par une membrane (16') élastique ou quasi rigide, qui agit en tant que paroi rigide de chambre, notamment en étant alimentée entièrement par un agent d'alimentation non compressible, notamment par un liquide (22) et en étant munie, le cas échéant, d'un moyen de limitation de l'excursion.

6. Dispositif médical de pompage suivant l'une des revendications 1 à 5, **caractérisé en ce que** son entraînement est disposé sensiblement dans une partie (6) d'entraînement pouvant être détachée du dispositif.

7. Dispositif médical de pompage suivant l'une des revendications 1 à 6, **caractérisé en ce qu'**il est prévu des éléments (30 à 34) d'étanchéité élastiques, tant dans la partie de l'extrémité du canal de l'élément (19) tournant de la vanne (13) sur celui-ci qu'également dans la partie des ouvertures (14, 17, 21) du corps (18) sur celui-ci.

8. Système médical de pompage comprenant un goutte-à-goutte (1) de perfusion ou un récipient (8, 9) de perfusion, ainsi qu'un dispositif de pompage suivant l'une des revendications (1 à 7) communiquant avec sa sortie.
